# EUROPEAN PATENT APPLICATION

(11) **EP 3 957 346 A1**
(43) Date of publication of application: **23.02.2022**
(21) Application number: 20191352.2
(22) Date of filing: 17.08.2020
(51) Int. Cl.: A61M 11/00, A61M 11/02, B01D 45/10, G01N 15/02

(54) **METHOD AND NEBULIZER FOR GENERATING AN AEROSOL HAVING AN ADJUSTABLE PARTICLE SIZE DISTRIBUTION**

(71) Applicant: Fraunhofer Gesellschaft zur Förderung der angewandten Forschung e.V., 80686 München (DE)
(72) Inventor: Clement, Alexander, 80686 München (DE); Pohlmann, Gerhard, 80686 München (DE)
(74) Representative: Altmann Stößel Dick Patentanwälte PartG mbB

(57) **Abstract**

The present invention relates to a method (150) and to a nebulizer (110) for generating an aerosol (112) having an adjustable particle size distribution (160). Further, the present invention relates to a respirator for distributing the aerosol (112) having a desired particle size (160) to a human being. Herein, the method (150) comprises the following steps:
a) guiding at least a portion of a stream (116) of a carrier gas (118) to a first wettable surface (120), wherein the first wettable surface (120) comprises a first liquid film (122), whereby an aerosol stream (124) is generated by detaching liquid particles (126) from the first liquid film (122);
b) depositing a first portion of the liquid particles (126) from the aerosol stream (124) onto a further wettable surface (128), whereby a further liquid film (130) is formed on the further wettable surface (128);
c) detaching a further portion of the liquid particles (126) from the further liquid film (130) formed on the further wettable surface (128) back to the aerosol stream (124);
d) repeating steps b) and c) until the adjustable particle size distribution (160) is obtained.

The method (150) and the nebulizer (110) are capable of generating liquid particles (126) having a mass median aerodynamic diameter (MMAD) of 2 µm or below, preferably of approx. 1.5 µm, thus allowing an application of the aerosol (112) also to babies, neonates and preterm neonates.

## Description

### Field of the invention

The present invention relates to a method and to a nebulizer for generating an aerosol having an adjustable particle size distribution. Further, the present invention relates to a respirator for distributing the aerosol having a desired particle size to a human being of any age, including elderly people, adults, children, infants, babies, neonates and preterm neonates, or to an animal.

### Related art

Pulmonary or respiratory diseases, including but not limited to asthma or chronic obstructive pulmonary disease (COPD), but also systemic diseases can be treated by an inhalation of drugs which are provided as liquid or solid particles in an aerosol stream. Herein, the particles which are generated by using an aerosol generator, preferably, exhibit a size that they become respirable. For this purpose, a mass median diameter (MMD) or mass median aerodynamic diameter (MMAD) can be used as a measure of a median particle size since the generated particles in the aerosol stream can be considered as polydisperse. Herein, particles for a respiration of applied drugs to adults, typically, exhibit an MMAD of approx. 3 µm, whereas infants, babies, neonates and preterm neonates, require smaller particles having an MMAD of approx. 1.5 µm only. As a result, it would be desirable to have a method and a nebulizer for generating an aerosol assuming an adjustable particle size distribution, thus, being able to satisfy such kinds of need in respiration.

In general, a nebulizer is used for generating an aerosol comprising at least one carrier gas, wherein the carrier gas carries a plurality of particles, wherein the plurality of particles comprises at least one of liquid particles or solid particles. For this purpose, the nebulizer comprises a reservoir designed for storing a liquid solution or a suspension, such as of a drug, which is supplied to an aerosol generator. According to the state of the art, the nebulizer may be selected from one of three main types, in particular from a pneumatic jet nebulizer, ultrasonic nebulizer, or a vibration nebulizer. However, each of these main types of nebulizers require an external energy source designated for providing energy, especially electric or pneumatic energy, while a size of the generated particles is, generally, limited to an MMAD of approx. 2 µm to 2.5 µm or more.

Recently, nebulizers which do not require an external energy source have been presented, for example SoftBreezer^{®} by URSATEC GmbH or Aqueous Droplet Inhaler by Pharmaero. Herein, both nebulizers use nozzle plates provided by MedSpray, Enschede, Netherlands, comprising micro- and nanotechnology processed spray nozzles having orifices of 1 to 30 µm only. These types of spray nozzles can be used for generating fine mists from fine liquid jets by applying the Rayleigh breakup mechanism. Such kinds of nozzle plates may, however, exhibit the disadvantage that liquid particles having a considerably high exit velocity are generated for which a high liquid pressure is required and by which high shear forces can be induced.

WO 98/01228 A2 discloses atomizing devices that form droplets through the Rayleigh breakup mechanism. Various embodiments include one or more of the following features. Liquid orifices form jets of liquid that form droplets. Gas orifices provide gas coflow that inhibits coalescence of the droplets. The liquid orifices can have non-circular cross-sectional shapes to promote Rayleigh breakup. Fluidic oscillators can also be provided to promote Rayleigh breakup. Supply networks are provided to supply gas and liquid to the gas and liquid orifices, respectively.

WO 99/33505 A1 discloses a particle separator for separating large particles of an inhalable active-particle/carrier gas flow with a housing having an inlet opening and an outlet opening. Further, the particle separator comprises a plurality of partition walls arranged behind each other between the inlet opening and the outlet opening in the housing. Each partition wall comprises at least one opening, wherein the openings of adjacent partition walls are arranged in a mutually displaced relationship and the partition walls are arranged at inclined positions to the longitudinal direction of the particle separator.

WO 2002/018058 A1 discloses a nozzle device and nozzle for atomization and/or filtration as well as methods for using the same, in particular to a micro-machined reinforced nozzle plate, that may produce small liquid droplets in air (spray) or into a liquid (emulsion) with a narrow droplet size distribution and to make small air bubbles into a liquid (foam) and to methods of making the same. Further a nozzle part for filtration as well as means and methods to facilitate atomization and filtration are disclosed.

WO 2009/045866 A1 discloses low flow rate cascade impactors for sampling aerosols, notably but not limited to pharmaceutical aerosols. The impactor stages serve as both orifice plate and collecting cup, simplifying collection and analysis. The impactor is designed to operate at flow rates approximating the inspiratory flow rates of young children and infants. Also presented is a method of and apparatus for applying a coating material to the collection surface of the stages after an impactor is assembled for use. The method entails generation of a polydisperse aerosol and sampling into the impactor. The coating substance improves the trapping of particles on the stages. The apparatus and method of application limit the amount of coating material applied and confines it to the regions of particle impact opposite the stage orifices.

Further, the Respimat^{®} provided by Boehringer Ingelheim is a further nebulizer which does not require an external energy source. Herein, a collision of miniature liquid jets is used for generating liquid particles. As disadvantages thereof, high shear forces are also induced here while solid particles can cause clogging in the nebulizer, thus requiring a sophisticated filtering technology.

WO 2004/058218 A2 discloses a method of forming droplets comprises flowing a liquid through a channel; spreading the liquid into a thin film in the channel; and impinging the thin film with a flowing gas to atomize the liquid into droplets. The droplets may then be dried to produce dried particles, such as particles comprising a pharmaceutical active agent. The droplets formed by the disclosed process have improved size and/or size distribution characteristics.

WO 2011/086552 A1 discloses a method and an apparatus for producing fine and concentrated aerosol from liquids, for various applications, by using rigid porous material, storage and easy streaming of the aerosol, (i.e. for inhalation), fast replacement of liquid carrier device and accuracy of output sprayed dose.

### Problem to be solved

It is therefore an objective of the present invention to provide a method and a nebulizer for generating an aerosol having an adjustable particle size distribution which could be able to satisfy different kinds of need in respiration, thereby avoiding the disadvantages and shortcomings of known methods and nebulizers.

In particular, it is desirable to be able to distribute an aerosol having the adjustable particle size to a human being of any age, including elderly people, adults, children, infants, babies, neonates and preterm neonates, or to an animal, preferably in a continuous fashion. More particular, it is desirable to provide a method and a nebulizer capable of generating liquid particles having a mass median diameter or MMAD of 2 µm or below, preferably of approx. 1.5 µm, without requiring an external energy source.

### Summary of the invention

This problem is solved by a method and a nebulizer for generating an aerosol having an adjustable particle size distribution as well as by a respirator for distributing an aerosol having an adjustable particle size to a human or to an animal being with the features of the independent claims. Preferred embodiments, which might be realized in an isolated fashion or in any arbitrary combination are listed in the dependent claims.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

In a first aspect, the present invention refers to a method for generating an aerosol having an adjustable particle size distribution. As generally used, the term "aerosol" refers to particles being suspended in air, wherein the particles may be selected from at least one of solid particles or liquid particles. The particles may, in particular, be or comprise particles of a pharmaceutical preparation, such as, for example, a lung surfactant. As further generally used, the term "generating an aerosol" relates to converting a liquid or a solid into an aerosol by using an aerosolizable material, i.e. a powder or a liquid solution, in an "aerosol generator", also denoted as "aerosolization device". According to the present invention, the aerosol preferably comprises liquid particles being generated by using a liquid solution.

The particles in the aerosol are, preferably, distributed across the carrier gas, in particular in a uniform and finely dispersed form. As a result, the aerosol is provided as an "aerosol stream" in which the aerosol particles are borne and/or carried by the carrier gas stream. Herein, the term "particle size distribution" refers to a function which describes a relative amount of particles for at least one particle size, especially for a particular particle size or for a range of particle sizes. Especially, a mass median diameter (MMD) or, preferably, a mass median aerodynamic diameter (MMAD) can be used for this purpose as an indication of the particle size since the particles in the aerosol stream can be considered as polydisperse.

As further indicated above, it may be advantageous to generate an aerosol having an adjustable particle size which could, thus, be applicable to a human being of any age, including elderly people, adults, children, infants, babies, neonates and preterm neonates, or to an animal. Herein, the particles for a respiration of applied drugs to adults, typically, exhibit an MMAD of approx. 3 µm, whereas infants, babies, neonates and preterm neonates, require smaller particles having an MMAD of approx. 1.5 µm only. Thus, it may be advantageous to have an adjustable particle size distribution that reflects a particular application of the method and to a related nebulizer for generating an aerosol.

The method for generating an aerosol having an adjustable particle size distribution according to the present invention comprising the following steps:
a) guiding at least a portion of a stream of a carrier gas to a first wettable surface, wherein the first wettable surface comprises a first liquid film, whereby an aerosol stream is generated by detaching liquid particles from the first liquid film;
b) depositing a first portion of the liquid particles from the aerosol stream onto a further wettable surface, whereby a further liquid film is formed on the further wettable surface;
c) detaching a further portion of the liquid particles from the further liquid film formed on the further wettable surface back to the aerosol stream;
d) repeating steps b) and c) until the adjustable particle size distribution is obtained.

Herein, a single cycle of the present method can, preferably, be performed with a particular amount of carrier gas in an order as indicated above, starting with step a) and consecutively repeating steps b) and c) in the given order according to step d), wherein a further amount of carrier gas can be provided according to step a) by which a further cycle can be initiated. Alternatively, the carrier gas can be provided in form of a continuous carrier stream and, similarly, treated in the order as indicated above, starting with step a) and consecutively repeating steps b) and c) in the given order according to step d), by which manner a constant aerosol stream can be generated.

According to step a), a stream of carrier gas is provided as a particular amount, such as a series of single pulses, or, as a preferred alternative, in a continuous fashion. As generally used, the term "carrier gas" refers to a gaseous composition which is designated for receiving and transporting an amount of liquid particles by which the desired aerosol stream is generated. For the purposes of the present invention, the terms "is provided" or "providing" refer to supplying a carrier gas which may, preferably, be selected from at least one of: ambient air; ambient air enriched by at least one further gas, in particular additional oxygen; and ambient air comprising liquid or solid particles from a different source as described below in more detail. However, particularly depending on the desired application, a different gaseous composition may also be feasible as the carrier gas.

Independently from the actual fashion of providing the aerosol stream, at least a portion of the stream of the carrier gas is guided to a first wettable surface, wherein the first wettable surface comprises a first liquid film. As generally used, the term "surface" relates to an area of a body which is, in accordance with the present invention, arranged in a manner that the stream of gas may be capable of impinging the area. Further, the term "wettable surface" indicates that the area of the body which is further designed for providing contact between the area and a liquid film attached to the area, preferably without any further chemical reaction between the liquid as comprised by the film and the area. As generally used, the term "liquid film" refers to a liquid substance which is provided in form of an extended structure having an extension across the surface which, typically, exceeds a thickness of the structure substantially perpendicular to an extension of the structure by a factor of at least 10, preferred of at least 100, more preferred of at least 1000, in particular at least 10.000. Herein, a balance is maintained within the liquid film between, on one hand, adhesive forces between the liquid substance and the surface which causes the liquid substance to spread across the surface, thereby forming the desired liquid film, and, on the other hand, cohesive forces within the liquid substance which aim to form at least one drop comprising a portion of the liquid which prefers separation from the surface. As indicated above, the "liquid substance" as used herein may comprise a solution, such as an aqueous solution or a nonaqueous solution, of at least one drug, specifically provided for treatment of at least one human being or animal.

Thus, at least a portion of the stream of the carrier gas is guided to the first wettable surface comprising a first liquid film. As further generally used, the terms "is guided" or "guiding" refer to leading the carrier gas in a manner that the liquid film is impinged by at least a portion of the carrier gas or, alternatively or in addition, in a manner that at least a portion of the carrier gas closely passes by the liquid film. As a result, the desired aerosol stream is generated by detaching liquid particles from the first wettable surface or, alternatively or in addition, by interrupting the carrier gas closely passing by the liquid film on sharp edges, whereby the generated aerosol stream comprises aerosol particles which are provided in this fashion by this particular particle source. As used herein, the term "first" with respect to a particular object indicates that at least one further object of the same kind or a similar kind may exist which can be used for the purposes of the present invention, wherein the at least one further object can be denoted by a term such as "further". By way of example, the term "first wettable surface" refers to the wettable surface which is, initially, impinged on by the carrier gas as defined above but which, prior to this particular impingement, does not comprise any liquid particles originating from the first liquid film, whereas the term "further wettable surface" refers to a wettable surface which is, thereafter, being impinged by a portion of the aerosol stream that has already been charged with liquid particles from the first liquid film. Similar distinctions may be made between a "first portion" of the liquid particles and a "further portion" of the liquid particles.

According to step b), a first portion of the liquid particles is deposited from the aerosol stream onto a further wettable surface. As used herein, the terms "is deposited" or "depositing" relate to transporting a portion of the liquid particles as comprised by the aerosol stream to an area on the further wettable surface in which this manner a further liquid film is formed on the further wettable surface. In particular, the depositing of the first portion of the liquid particles from the aerosol stream onto the further wettable surface may, preferably, comprise an impact of a part of the first portion of the liquid particles onto the further wettable surface.

According to step c), a further portion of the liquid particles is detached from the further liquid film formed on the further wettable surface back to the aerosol stream. As used herein, the terms "is detached" or "detaching" refer to removing a further portion of the liquid particles from the further film and transporting those liquid particles back to the aerosol stream that passes the area on which the further film is located on the further wettable surface.

According to step d), the above-indicated steps b) and c) are repeated until the adjustable particle size distribution is obtained. Herein, a repetition of steps b) and c) may, preferably, be performed consecutively on a plurality of further wettable surfaces. As described below in more detail, the plurality of the further wettable surfaces may, in particular, be arranged in form of a cascade along a course of the aerosol stream, preferably, in a fashion that a part of the aerosol stream consecutively impinges on one of the further wettable surfaces, whereby, each time, step b) and step c) is executed on a different further wettable surface. As generally used, the term "course" relates to a direction of flow of the aerosol stream, preferably along the plurality of the further wettable surfaces or a portion thereof. As further generally used, the term "cascade" refers to a consecutive arrangement of further wettable surfaces in a fashion that at least a part of the aerosol stream can consecutively pass along any one of the further wettable surfaces or portions thereof. In accordance with the present invention the aerosol stream may, preferably, pass along at least 2, 3, 4, 5, 6, 7, 8, 10, 12 or 15 up to 20, 25, 30, 40 or 50 individual wettable surfaces. However, a different number of individual wettable surfaces may also be feasible.

Not wishing to be bound by theory, a deposition rate of the liquid particles, in general, depends on a size of the liquid particles, wherein, under normal circumstances, the deposition rate increases with increasing size of the liquid particles. As generally used, the term "deposition rate" refers to a ratio of particles which are subject to deposition on a particular area. As a result thereof, the impact of a part of the liquid particles that have a larger size from the aerosol stream onto a further wettable surface is preferred. Thus, a reduction of an average particle size of the liquid particles in the aerosol stream may be achieved after the liquid particles that have a larger size are more likely to be deposited from the aerosol stream onto the further wettable surface. Consequently, the particle size distribution of the further portion of the liquid particles can, therefore, be shifted to smaller particle sizes compared to the particle size distribution of the first portion of the liquid particles.

In order to arrive at the desired adjustable particle size distribution, the skilled person can, based on common knowledge about aerosol streams and, optionally, supported by corresponding simulations, easily adjust at least one parameter which is related to the wettable surfaces and respective corresponding arrangements. For this purpose, at least one parameter, in particular at least one geometric parameter, related to the further wettable surface may, preferably, be adjusted in accordance with the known course of the aerosol stream and/or in order to modify the course of the aerosol stream, especially for optimizing the impact of the part of the liquid particles onto the further wettable surfaces. As generally used, the term "parameter" refers to a representation of an input of a feature of the further wettable surface to the resulting particle size distribution. Specifically, the at least one geometric parameter related to the further wettable surface may, thus, be selected from at least one of: a distance between adjacent further wettable surfaces, an incident angle of the further wettable surface with respect to a course of the aerosol stream, a curvature of a wettable surface. However, further kinds of geometric parameters may also be feasible.

Alternatively or in addition, at least one parameter which is related to a wetting property of the further wettable surface may be adjusted in order to influence the resulting particle size distribution within the aerosol stream. As generally used, the term "wetting property" refers to at least one mechanical, physical, or chemical property of the wettable surface which is designated for carrying the liquid film and which is, thus, capable of influencing a dependence of the deposition rate of the liquid particles on the size of the liquid particles. Consequently, the skilled person can, based on common knowledge about mechanical, physical, or chemical properties of typical materials which are used for such a purpose and, optionally, supported by simple experimental verifications, easily select a material for the wettable surface and/or modify at least one area on the surface thereof, in particular a roughness of the surface thereof, specifically by employing an additive or a subtractive treatment, such as milling, etching, or depositing, wherein the depositing may, preferably, comprise a process selected from chemical vapor deposition (CVD) or atomic layer deposition (ALD). However, further kinds of deposition processes may also be conceivable. Alternatively or in addition, the surface of the further wettable surface may comprise at least one of a microstructure or a nanostructure. As generally used, the term "microstructure" refers to a structuring of the surface comprising grooves or protrusions which exhibit an extension, such as a width, a height, or a depth, respectively, in the range of 1 µm to less than 1000 µm, preferably of 10 µm to 100 µm. Similarly, the term "nanostructure" refers to a structuring of the surface comprising grooves or protrusions which exhibit an extension, such as a width, a height, or a depth, respectively, in the range of 1 nm to less than 1000 nm, preferably of 10 nm to 100 nm. However, further kinds of parameters related to a wetting property of the wettable surface may also be feasible.

In particular contrast to WO 2011/086552 A1 which discloses a method and an apparatus for producing aerosol from liquids by using a rigid porous material which acts as a pneumatic multi-nozzle atomizing system, wherein the porous medium is considered as an integral system comprising a liquid, located on the surface of the porous medium and/or partially absorbed in it, for spraying; a large number of pores of sub-micron size acting as nozzles; and gas caged in the pores which are vacant of liquid, the present invention employs a plurality of non-porous wettable, surfaces designated for consecutively depositing particles from at least a part of the aerosol stream and detaching particles from the liquid film.. As a consequence, each area used as a wettable surface is, particularly preferred, devoid of any pores, especially in order to avoid as far as possible a disturbance of the repeated process of depositing and detaching of the liquid particles on the wettable surfaces, by which process the particle size distribution is modified as described elsewhere herein.

In a further aspect, the present invention relates to a nebulizer for generating an aerosol having an adjustable particle size distribution. As generally used, the term "nebulizer" refers to a device which is used for generating an aerosol comprising at least one carrier gas, wherein the carrier gas carries a plurality of liquid or solid particles. As indicated above, the aerosol according to the present invention preferably comprises liquid particles being generated by using a liquid solution and a carrier gas.

In accordance with the present invention, the nebulizer comprises:
- at least one inlet designated for receiving a stream of a carrier gas;
- a first wettable surface comprising a first liquid film designated for generating an aerosol stream by receiving at least a portion of the stream of the carrier gas and by detaching liquid particles from the first liquid film;
- a plurality of further wettable surfaces, wherein each further wettable surface is designated for
   ∘ depositing a first portion of the liquid particles from the aerosol stream for forming a further liquid film on the further wettable surface, and
   ∘ detaching a further portion of the liquid particles from the further liquid film formed on the further wettable surface back to the aerosol stream;
- at least one outlet designated for providing the aerosol having the adjustable particle size distribution.

Thus, the nebulizer exerts a function of a "nozzle" having an inlet and an outlet, wherein the carrier gas as provided is introduced by the inlet while the aerosol as generated is provided by the outlet, wherein the liquid particles are generated from a first liquid film comprising a liquid solution, such as of a drug, deposited on a first wettable surface, wherein the adjustable particle size distribution, particularly shifted to smaller particle sizes, is provided by consecutively depositing and detaching liquid particles to and from a plurality of further wettable surfaces, preferably arranged along a course of at least a part of the aerosol stream, in a fashion that each further wettable surface is designated for reducing an average particle size. In general, the nebulizer as disclosed herein, may be used in pressurized metered dose inhalers (pMDIs), dry powder inhalers (DPIs) and soft mist inhalers (SMIs).

In a further aspect, the present invention relates to a respirator for distributing an aerosol having an adjustable particle size to a human being or to an animal. As indicated above, the human being can be of any age and, thus, includes elderly people, adults, children, infants, babies, neonates and preterm neonates. As generally used, the term "respirator" refers to a device which is designated for providing drug administration and/or ventilation support to a human being or to an animal by generating the aerosol and by administering it to an orifice of the human or the animal in a fashion that it becomes respirable, wherein the aerosol has the desired particle size for the particular human being or animal. Herein, the respirator may be applicable with ventilated applications, i.e. passive applications, and non-ventilated applications, i.e. active applications. As a result, the respirator may be applicable to both passive users respirated by stationary devices and active users employing handheld devices.

In accordance with the present invention, the respirator comprises:
- at least one generator designated for generating a carrier gas stream;
- at least one nebulizer for generating an aerosol having an adjustable particle size distribution as described elsewhere herein;
- at least one applicator for providing the aerosol having the adjustable particle size to a human being.

As generally used, the term "generator" refers to an arbitrary apparatus which is designated for generating a desired carrier gas stream. For this purpose, the generator could employ at least one of: ambient air readily available; a reservoir comprising at least one further gas, in particular additional oxygen for enriching ambient air; a different source as described herein for providing liquid or solid particles.

As further generally used, the term "applicator" refers to an arbitrary device which is designated for administering the aerosol as provided by the nebulizer to the human being or to the animal. For this purpose, the applicator, also denoted by "patient interface", may, in particular, be selected from a mouthpiece, a breathing mask, a nasal cannula or a tracheal cannula. However, other types of applicators may also be feasible.

In a further aspect, the present invention relates to a use of the respirator in providing drug administration and/or ventilation support to a human being or to an animal.

For further details referring to at least one of the nebulizer, the respirator, or the use thereof, reference may be made to the description above with respect to the method for generating an aerosol according to the present invention and to the detailed description of the embodiments below.

### Short description of the Figures

Further optional features and embodiments of the invention will be disclosed in more detail in the subsequent description of preferred embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the Figures. Therein, identical reference numbers in these Figures refer to identical or functionally comparable elements.

In the Figures:
- Figures 1 to 3:: schematically illustrate preferred exemplary embodiments of a nebulizer for generating an aerosol having an adjustable particle size distribution according to the present invention;
- Figure 4:: schematically illustrates a preferred exemplary embodiment of a method for generating an aerosol having an adjustable particle size distribution according to the present invention; and
- Figure 5:: presents a diagram schematically illustrating a shift of a particle size distribution of a further portion of the liquid particles to smaller particle sizes compared to the particle size distribution of a first portion of the liquid particles.

### Detailed description of the embodiments

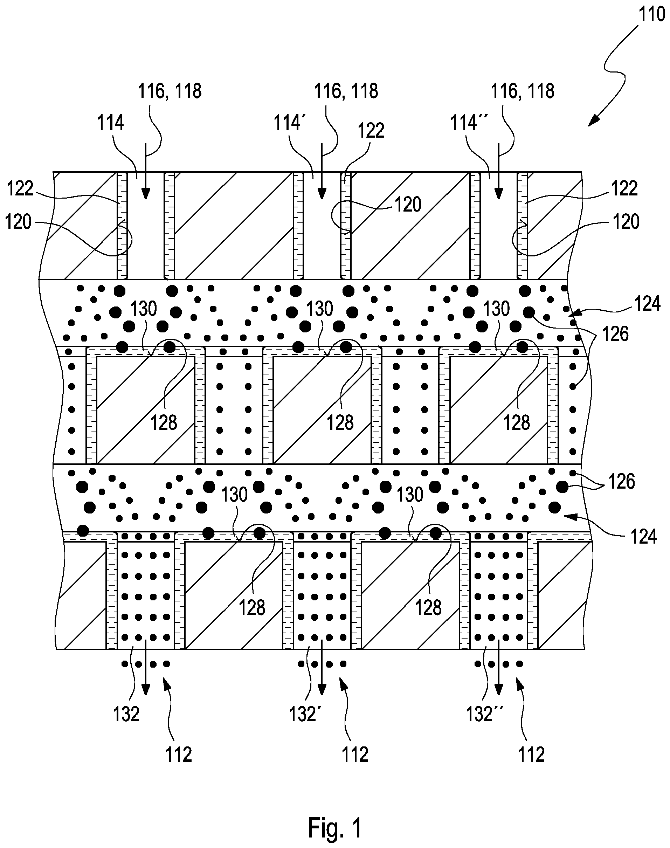

Figures 1 to 3 schematically illustrate preferred exemplary embodiments of a nebulizer 110 for generating an aerosol 112 having an desired particle size distribution according to the present invention.

As depicted in Figure 1, the nebulizer 110 for generating the aerosol 112 having the adjustable particle size distribution comprises inlets 114, 114', 114", wherein each inlet 114, 114', 114" is designated for receiving a stream 116 of a carrier gas 118. As described above, the carrier gas 118 may be provided by a generator (not depicted here), wherein the generator employs ambient air readily available or a reservoir comprising at least one further gas, in particular additional oxygen for enriching the ambient air. In the preferred exemplary embodiments of the nebulizer 110 as illustrated here, the stream 116 of the carrier gas 118 at each inlet 114, 114', 114" is devoid of any liquid or solid particles. However, in further embodiments (not depicted here), the stream 116 of the carrier gas 118 at one or more of the inlets 114, 114', 114" may comprise liquid or solid particles that may be provided by a different source as described herein.

As further illustrated in Figure 1, the nebulizer 110 according to the present invention comprises a first wettable surface 120, wherein a first liquid film 122 is deposited on the first wettable surface 120. As schematically depicted there, a portion of the stream 116 of the carrier gas 118 is guided to the first wettable surface 120 which comprises the first liquid film 122 in a manner that the portion of the carrier gas 118 closely passes by the liquid film 122, whereby the desired aerosol stream 124 is generated by detaching liquid particles from the first wettable surface 120. As a result, the aerosol 112 which is generated in this fashion comprises liquid aerosol particles 126 which are provided by the nebulizer 110 as particle source.

As further illustrated in Figure 1, the nebulizer 110 according to the present invention comprises a plurality of further wettable surfaces 128, wherein each further wettable surface 128 is designated for depositing a first portion of the liquid particles 126 from the aerosol stream 124 for forming a further liquid film 130 on the further wettable surface 128. In addition, each further wettable surface 128 is designated for detaching a further portion of the liquid particles 126 from the further liquid film 130 formed on the further wettable surface 128 back to the aerosol stream 124. As schematically depicted in Figure 1, a deposition rate of the liquid particles 126, generally, depends on a size of the liquid particles 126, wherein, under normal circumstances, the deposition rate increases with increasing size of the liquid particles 126. As a result thereof, the impact of a part of the liquid particles 126 that have a larger size from the aerosol stream 124 onto each further wettable surface 128 is preferred. Thus, a reduction of an average particle size of the liquid particles 126 in the aerosol stream 124 may be achieved after the liquid particles 126 that have a larger size are more likely to be deposited from the aerosol stream 124 onto each further wettable surface 128.

Consequently, the particle size distribution of the further portion of the liquid particles 126 can, therefore, be shifted to smaller particle sizes compared to the particle size distribution of the first portion of the liquid particles 126. Thus, both the depositing of the first portion of the liquid particles 126 from the aerosol stream 124 for forming the further liquid film 130 on the further wettable surface 128 and the detaching of the further portion of the liquid particles 126 from the further liquid film 130 formed on the further wettable surface 128 back to the aerosol stream 124 are repeated until the adjustable particle size distribution is obtained.

In accordance with the embodiment of the nebulizer 110 as shown in Figure 1, the plurality of the further wettable surfaces 128 is arranged in form of a cascade, thus generating a course of the aerosol stream 124, thus, allowing the aerosol stream 124 consecutively impinging on or passing by one of the further wettable surfaces 128, whereby, each time, the depositing of the first portion of the liquid particles 126 from the aerosol stream 124 and the detaching of the further portion of the liquid particles 126 from the further liquid film 130 is executed on one of the different further wettable surfaces 128.

In order to obtain the desired adjustable particle size distribution, the skilled person can, based on common knowledge about aerosol streams and, optionally, supported by corresponding simulations, easily adjust at least one parameter particularly selected from at least one geometric parameter and at least one parameter to a wetting property of the wettable surfaces 120, 128. Specifically, the at least one geometric parameter may be selected from at least one of: a distance between adjacent further wettable surfaces 128, or an incident angle of the wettable surfaces 120, 128 with respect to a course of the aerosol stream 124.

Further, the at least one parameter related to a wetting property of the wettable surfaces 120, 128 may be selected from at least one of a wettability of a material used for this purpose or, alternatively or in addition, a surface modification of the wettable surfaces 120, 128, in particular a roughness of the wettable surfaces 120, 128, specifically obtained by applying an additive or a subtractive treatment, such as milling or etching the wettable surfaces 120, 128, or depositing at least one layer. However, further kinds of parameters may also be feasible. As indicated above, each wettable surface 120, 128 is, particularly preferred, devoid of any pores, especially in order to avoid as far as possible a disturbance of the repeated process of depositing and detaching of the liquid particles 126 on the wettable surfaces 120, 128, by which process the particle size is distribution is modified as described above or below in more detail.

As further depicted in Figure 1, the nebulizer 110 for generating the aerosol 112 comprises outlets 132, 132', 132", wherein each outlet 132, 132', 132" is designated for providing the aerosol 112 having the adjustable particle size distribution, in particular, to an applicator (not depicted here) designated for administering the aerosol 112 as provided by the nebulizer 110 to a human being or to an animal which may, especially, be selected from a mouthpiece, a breathing mask, a nasal cannula or a tracheal cannula. However, other arrangements for the outlets or a different type of applicator may also be feasible.

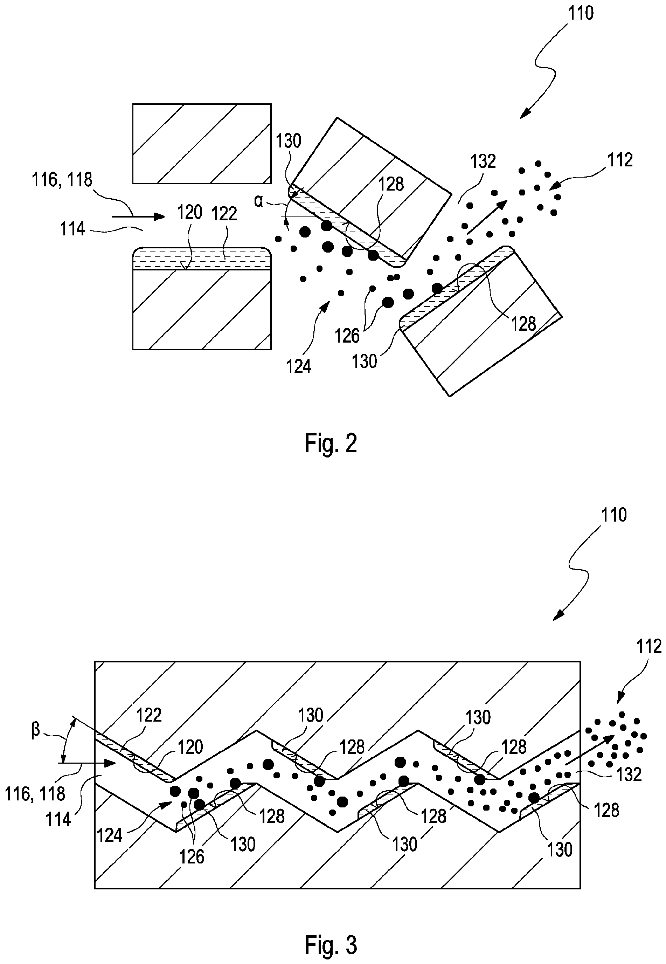

The further preferred exemplary embodiments of the nebulizer 110 as illustrated in Figures 2 and 3 differ from the exemplary embodiment of Figure 1 as described above, mainly, by an arrangement of the wettable surfaces 120, 128 with respect to each other and with respect to a direction of the stream 116 of the carrier gas 118 and the aerosol 112, respectively. Whereas the further wettable surfaces 128 are arranged perpendicular to a main direction of the aerosol stream 124 in Figure 1, the further wettable surfaces 128 as schematically depicted in Figure 1 assume an angle 30° ≤ α ≤ 60° with regard to the main direction of the aerosol stream 124 in Figures 2 and 3. Further, the direction of the stream 116 of the carrier gas 118 is approximately parallel to the first wettable surface 120 in Figures 1 and 2, while an angle between the direction of the stream 116 of the carrier gas 118 and the first wettable surface 120 assumes β ≈ 30° as schematically depicted in Figure 3, thus, allowing the first liquid film 122 to be impinged by at least a portion of the carrier gas 118 in order to increase the number of aerosol particles 126 which are provided by this particular arrangement. However, further values for the angles α, β (not depicted here) may also be feasible. For further details, reference may be made to the description of the nebulizer as described above with regard to Figure 1.

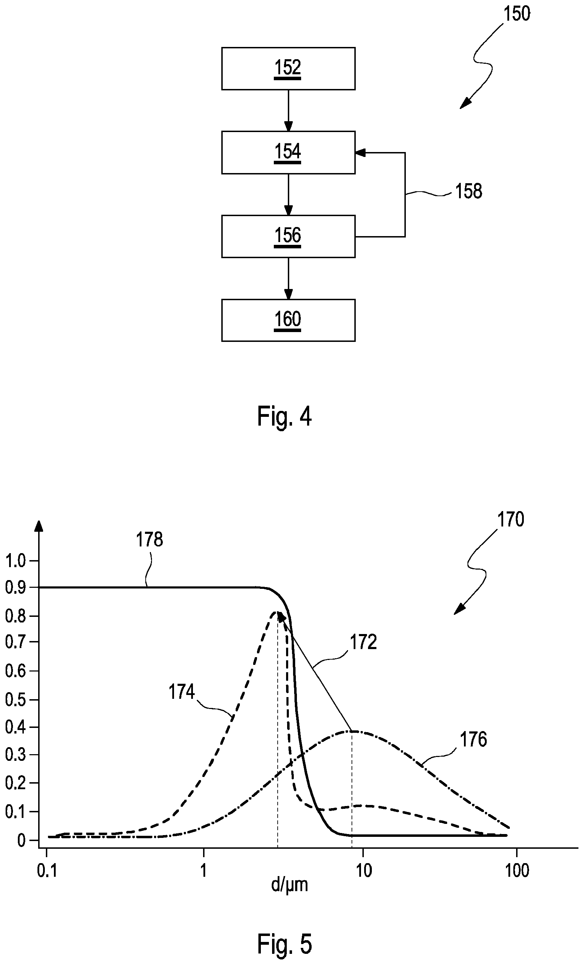

Figure 4 schematically illustrates a preferred exemplary embodiment of a method 150 for generating an aerosol 112 having an adjustable particle size distribution according to the present invention.

According to a providing step 152, at least a portion of the stream 116 of the carrier gas 118 is guided in accordance with step a) to the first wettable surface 120 which comprises the first liquid film 122, whereby, as described above in more detail, the aerosol stream 124 is generated by detaching liquid particles from the first liquid film 122.

According to a depositing step 154, the first portion of the liquid particles 126 is deposited in accordance with step b) from the aerosol stream 124 onto the further wettable surface 128, whereby the further liquid film 130 is formed on the further wettable surface 130.

According to a detaching step 156, the further portion of the liquid particles 126 is detached in accordance with step c) from the further liquid film 128 formed on the further wettable surface 130 back to the aerosol stream 130.

According to a repeating step 158, the depositing step 154 and the detaching step 156 are repeated in accordance with step d) until the adjustable particle size distribution is obtained as indicated by reference sign 160.

For further details regarding the method 150 for generating the aerosol 112 according to the present invention, reference may be made to the description of Figures 1 to 3 above.

Figure 5 presents a diagram 170 which schematically illustrates an occurrence of a shift 172 to smaller particle sizes of a particle size distribution 174 related to the further portion of the liquid particles 126 after the detaching step 156 compared to a particle size distribution 176 of the first portion of the liquid particles 126 prior to the depositing step 154. Accordingly, the occurrence of the shift 172 can be explained by assuming that the particle size distribution 174 related to the further portion of the liquid particles 126 can be considered as a convolution of the particle size distribution 176 of the first portion of the liquid particles 126 with a curve 178 of the liquid particles 126 with respect to a particle size d/µm, wherein the curve 178 shows values for 1 - deposition rate.

In the simulation as depicted in Figure 5, a mass median diameter (MMAD) of 9 µm has been assumed for the particle size distribution 176 of the first portion of the liquid particles 126, whereas the MMAD of the particle size distribution 174 related to the further portion of the liquid particles 126 amounts to approx. 3 µm after using 7 further wettable surfaces 130. Further simulations are feasible.

### List of reference numbers

- 110: nebulizer
- 112: aerosol
- 114: inlet
- 116: stream
- 118: carrier gas
- 120: first wettable surface
- 122: first liquid film
- 124: aerosol stream
- 126: liquid particles
- 128: further wettable surface
- 130: further liquid film
- 132: outlet
- 150: method
- 152: providing step
- 154: depositing step
- 156: detaching step
- 158: repeating step
- 160: adjustable particle distribution
- 170: diagram
- 172: shift
- 174: particle size distribution related to the further portion of the liquid particles
- 176: particle size distribution related to the first portion of the liquid particles
- 178: curve showing 1 - deposition rate

## Claims

1. A method (150) for generating an aerosol (112) having an adjustable particle size distribution (160), the method (150) comprising the following steps:
a) guiding at least a portion of a stream (116) of a carrier gas (118) to a first wettable surface (120), wherein the first wettable surface (120) comprises a first liquid film (122), whereby an aerosol stream (124) is generated by detaching liquid particles (126) from the first liquid film (122);
b) depositing a first portion of the liquid particles (126) from the aerosol stream (124) onto a further wettable surface (128), whereby a further liquid film (130) is formed on the further wettable surface (128);
c) detaching a further portion of the liquid particles (126) from the further liquid film (130) formed on the further wettable surface (128) back to the aerosol stream (124);
d) repeating steps b) and c) until the adjustable particle size distribution (160) is obtained.

2. The method (150) according to the preceding claim, wherein depositing the first portion of the liquid particles (126) from the aerosol stream (124) onto the further wettable surface (128) comprises an impact of a part of the first portion of the liquid particles (126) onto the further wettable surface (128).

3. The method (150) according to any one of the preceding claims, wherein the depositing of the first portion of the liquid particles (126) from the aerosol stream (124) onto the further wettable surface (128) comprises reducing an average particle size of the first portion of the liquid particles (126) after deposition on the further wettable surface (128).

4. The method (150) according to any one of the preceding claims, wherein a particle size distribution (174) of the further portion of the liquid particles (126) is shifted to smaller particle sizes compared to the particle size distribution (176) of the first portion of the liquid particles (126).

5. The method (150) according to any one of the preceding claims, wherein repeating steps b) and c) is performed consecutively on a plurality of further wettable surfaces (128).

6. The method (150) according to any one of the preceding claims, wherein the adjustable particle size distribution (160) is obtained by adjusting at least one of: at least one geometric parameter related to the further wettable surface (128), at least one parameter related to a wetting property of the further wettable surface (128).

7. The method (150) according to the preceding claim, wherein the at least one of the geometric parameter relates to at least one of: a distance between adjacent further wettable surfaces (128), an incident angle of at least one of the further wettable surfaces (128) with respect to a course of the aerosol stream (124).

8. A nebulizer (110) for generating an aerosol (112) having an adjustable particle size distribution (160), the nebulizer (110) comprising
- at least one inlet (114, 114', 114") designated for receiving a stream (116) of a carrier gas (118);
- a first wettable surface (120) comprising a first liquid film (122) designated for generating an aerosol stream (124) by receiving at least a portion of the stream (116) of the carrier gas (118) and by detaching liquid particles (126) from the first liquid film (122);
- a plurality of further wettable surfaces (128), wherein each further wettable surface (128) is designated for
∘ depositing a first portion of the liquid particles (126) from the aerosol stream (124) for forming a further liquid film (130) on the further wettable surface (130), and
∘ detaching a further portion of the liquid particles (126) from the further liquid film (130) formed on the further wettable surface (128) back to the aerosol stream (124);
- at least one outlet (132. 132', 132") designated for providing the aerosol (112) having the adjustable particle size distribution (160).

9. The nebulizer (110) according to the preceding claim, wherein each further wettable surface (128) is designated for receiving the first portion of the liquid particles (126) from the aerosol stream (124) in form of an impact of a part of the first portion of the liquid (126) particles onto the further wettable surface (128).

10. The nebulizer (110) according to the preceding claim, wherein each further wettable surface (128) is designated for reducing an average particle size of the first portion of the liquid particles (126) by depositing the liquid particles (126) having a larger size on the further wettable surface (128) and detaching the liquid particles (126) from the further wettable surface (128).

11. The nebulizer (110) according to any one of the preceding claims referring to a nebulizer (110), wherein the plurality of the further wettable surfaces (128) is arranged along a course of at least a portion of the aerosol stream (124).

12. The nebulizer (110) according to any one of the preceding claims referring to a nebulizer (110), wherein the plurality of the further wettable surfaces (128) is arranged in a manner that a part of the aerosol stream (124) consecutively impinges on one of the further wettable surfaces (128).

13. The nebulizer (110) according to any one of the preceding claims referring to a nebulizer (110), wherein at least one geometric parameter related to the further wettable surface (128) and at least one parameter related to a wetting property of the further wettable surface (128) is adjusted in a manner that the adjustable particle size distribution (160) is obtained.

14. The nebulizer (110) according to the preceding claim, wherein the at least one of the geometric parameter relates to at least one of: a distance between adjacent further wettable surfaces (128), an incident angle of at least one of the further wettable surfaces (128) with respect to a course of the aerosol stream (124).

15. A respirator for distributing an aerosol (112) having an adjustable particle size (160) to a human being or to an animal, the respirator comprising:
- at least one generator designated for generating a carrier gas stream;
- at least one nebulizer (110) according to any one of the preceding claims referring to a nebulizer (110) designated for generating an aerosol (112) having the adjustable particle size distribution (160);
- at least one applicator for providing the aerosol (112) having the adjustable particle size (160) to the human being or to the animal.
